# EUROPÄISCHE PATENTSCHRIFT

(11) **EP 0 488 378 B1**
(45) Veröffentlichungstag und Bekanntmachung des Hinweises auf die Patenterteilung: **20.05.1998**
(21) Anmeldenummer: 91120536.7
(22) Anmeldetag: 29.11.1991
(51) Int. Cl.: A61F 2/38

(54) **Kniegelenkendoprothese**
Knee endoprosthesis
Endoprothèse de genou

(30) Priorität: 29.11.1990 DE 4038037
(43) Veröffentlichungstag der Anmeldung: 03.06.1992
(60) Teilanmeldung: 95115227.1
(73) Patentinhaber: GMT Gesellschaft für medizinische Technik mbH, D-22767 Hamburg (DE); Waldemar Link GmbH & Co., 22339 Hamburg (DE)
(72) Erfinder: Engelbrecht, Eckart, W-2000 Hamburg 60 (DE); Nieder, Elmar, W-2155 Jork (DE); Keller, Arnold Karl Walter, 23863 Kayhude (DE)
(74) Vertreter: Heldt, Gert, Dr. Dipl.-Ing.

(56) Entgegenhaltungen:
- DE-A- 2 114 287
- DE-A- 2 122 390
- DE-A- 2 539 717
- DE-A- 3 119 841

## Beschreibung

Die Erfindung betrifft eine Kniegelenkendoprothese mit einem Femurteil und einem Tibiateil, die um ein sie miteinander verbindendes Scharniergelenk, dessen Schwenkachse einen nicht veränderbaren Abstand sowohl vom Femurteil als auch vom Tibiateil besitzt, von einer gestreckten Stellung in eine abgewinkelte Stellung in einer Beugeebene verschwenkbar gelagert sind und jeweils Schäfte aufweisen, die in abgewinkelter Stellung einen Beugewinkel zwischen sich einschließen und in gestreckter Stellung in einander entgegengesetzte Richtungen weisen, wobei das Femurteil an ihm befestigte bogenförmige Flügel aufweist und sich durch die Schäfte jeweils eine Längsachse erstreckt, die in gestreckter Stellung im wesentlichen in quer zur Beugeebene verlaufenden einander planparallelen Schaftebenen angeordnet sind, wobei von den Schaftebenen die des Femurteils einen größeren Horizontalabstand von einer planparallel zu ihnen durch die Schwenkachse verlaufende Vertikalebene in Richtung auf einen vom Beugewinkel eingeschlossenen hinteren Teil des Femurteils einhält als die Schaftebene des Tibiateils und wobei zwischen der Schaftebene des Tibiateils und der Schaftebene des Femurteils ein Schaftabstand besteht und eine senkrecht zur Vertikalebene durch die Schwenkachse verlaufende Horizontalebene in Richtung auf den Tibiateil einen Vertikalabstand zu einer horizontalen Verschiebeebene, die sich tangential zu einer auf dem Tibiateil ausgebildeten Auflagefläche erstreckt, einhält.

Derartige Kniegelenkendoprothesen sind als Scharniergelenke in der Fachwelt bekannt und werden erfolgreich insbesondere dann implantiert, wenn sich herausstellt, daß bei einem zu behandelnden Patienten der das Kniegelenk umgebende Weichteilapparat nicht mehr stark genug ausgebildet ist, um den Femurteil mit der notwendigen Kraft im Wirkzusammenhang mit dem Tibiateil zu halten. Bei einer derartigen Diagnose ist auch der Ersatz des Kniegelenkes durch eine Endoprothese erfahrungsgemäß nicht möglich, bei der ein die Beugebewegungen der beiden Gelenkteile ermöglichendes Scharniergelenk durch konstruktive Maßnahmen in die Lage versetzt wird, Längsbewegungen entweder in Richtung eines Oberschenkelknochens oder eines Unterschenkelknochens durchzuführen und die Stabilität der gesamten Endoprothese dadurch erreicht wird, daß die beiden Prothesenteile einander formschlüssig beaufschlagen. Die Schädigung der Weichteilummantelung führt dazu, daß diese für die Stabilisierung eines solchen Systems nicht ausreicht, um Materialbelastungen des Systems oder Luxationen durch Verschiebungen des Gelenkes zu verhindern, die mit erheblichen Schmerzen für den Patienten verbunden sind und dazu führen, daß die luxierte Endoprothese funktionsunfähig wird.

Diese Schwierigkeiten stellen bei den Scharnierprothesen der einleitend genannten Art nur ein geringes Risiko dar, da sich bei ihnen der Abstand, der konstruktiv zwischen dem Scharniergelenk einerseits und dem Femurteil bzw. dem Tibiateil andererseits besteht, nicht verändern kann. Der Formvollschluß bringt eine erhöhte Stabilität in die Endoprothese und beinhaltet lediglich ein geringes Risiko für Materialschäden. Das Scharniergelenk sorgt mithin dafür, daß der Femurteil mit dem Tibiateil ständig in einer vorgegebenen Betriebsstellung zusammenwirken. Bewegungen des Femurteils bezüglich des Tibiateils bzw. des Tibiateils bezüglich des Femurteils in Längsrichtung weder des Unterschenkels- noch des Oberschenkelknochens sind möglich. Andererseits bewirkt diese feste Zuordnung des Tibiateils einerseits und des Femurteils andererseits durch das hinsichtlich seiner Lage unverschiebliche Scharniergelenk, daß sämtliche Schwenkbewegungen lediglich stattfinden können um die bezüglich der beiden Gelenkteile unverschiebliche und in ihrer Lage genau definierte Mittelachse des Schwenkgelenks. Diese Mittelachse kann sich daher veränderlichen Belastungszuständen und Lagen des Kniegelenkes nicht anpassen. Insoweit trägt ein solches Scharniergelenk den in einem natürlichen Kniegelenk ablaufenden Bewegungen nicht genügend Rechnung, sondern stellt einen Kompromiß hinsichtlich der gewünschten Betriebssicherheit und dem erzielbaren Bewegungsablauf dar. Bei einem natürlichen Kniegelenk wandert die Schwenkachse, um die der Unterschenkelknochen gegenüber dem Oberschenkelknochen verschwenkt wird, je nach Beugewinkel des Kniegelenkes und der jeweiligen Lage des Unterschenkelknochens in umschriebenen Grenzen.

Um eine möglichst natürliche Funktion und einen dauerhaften Sitz einer Kniegelenkendoprothese gewährleisten zu können, sind bisher eine Vielzahl von Wegen mit dem Ziel beschritten worden, die natürlichen Bewegungsabläufe so weitgehend wie möglich zu simulieren. Dabei wurde versucht, der Scharniergelenkachse eine zwischen dem Tibiateil und dem Femurteil liegende Lage zu vermitteln, die geeignet zu sein schien, eine vielzahl von Schwenkbewegungen des Tibiateils bezüglich des Femurteils zu ermöglichen, ohne daß deswegen durch eine starke Beanspruchung der jeweils benachbarten Knochenteile sich entweder der Tibiateil oder der Femurteil oder beide Teile in ihren jeweiligen Halterungen lösten. Auf diese Weise wurden Bedingungen für eine spezifische Lage der sogenannten Kompromißsachse in der Oberschenkelrolle erarbeitet und verwirklicht. Die bisher gefundenen Lösungen zeigen aber deutlich, daß eine befriedigende Lage die Kompromißachse bisher nicht gefunden wurde. Einerseits konnte bisher noch keine befriedigende Lösung dafür gefunden werden, durch eine wandernde Achse in einer Scharniergelenkkonstruktion Bedingungen herzustellen, die den natürlichen Bedingungen entsprechen. Andererseits treten bei den bisher bekannt gewordenen Scharniergelenken relativ häufig mechanische Beeinträchtigungen beispielsweise im Bereich des Scharniergelenks oder der Verankerung innerhalb der benachbarten Knochen auf. Die Beeinträchtigungen sind relativ häufig so umfangreicher Art, daß ein Ausbau der Scharniergelenkendoprothese sich als notwendig erweist und eine neue Prothese eingesetzt werden muß. Beim Einsatz neuer Prothesen werden im Regelfall Knochenresektionen relativ umfangreicher Art notwendig, von denen jede einzelne dazu führt, daß Knochenmasse verloren geht, die bei späteren Reoperationen fehlt.

Aus der DE-A-212 23 90 ist eine Kniegelenkendoprothese bekannt, die als Scharniergelenk ausgebildet ist, bei dem ein Femurteil über eine Welle um deren Schwenkachse schwenkbar mit einem Tibiateil verbunden ist. Durch die Schäfte verlaufen Längsachsen, die in gestreckter Stellung im wesentlichen in quer zur Beugeebene verlaufenden planparallelen Schaftebenen angeordnet sind. Die Schaftebene des Femurschaftes ist in der Schaftebene des Tibiaschaftes anterior vorgelagert. Die Schaftebene des Tibiaschaftes fällt etwa mit einer durch die Schwenkachse verlaufenden Vertikalachse zusammen. Mit dem Femurteil fest verbunden sind bogenförmige Flügel vorgesehen, die in Gleitpfannen des Tibiateils gleiten können. Eine horizontale Verschiebeebene verläuft tangential zu einer auf dem Tibiateil ausgebildeten Auflagefläche in einem vertikalen Abstand von einer durch die Schwenkachse verlaufenden Horizontalebene.

Aufgabe der vorliegenden Erfindung ist es daher, eine Kniegelenkendoprothese der einleitend genannten Art so zu verbessern, daß die mit ihrer Hilfe durchführbaren Schwenkbewegungen weitgehend den Schwenkbewegungen eines natürlichen Kniegelenkes angepaßt sind.

Diese Aufgabe wird erfindungsgemäß dadurch gelöst, daß der Horizontalabstand zwischen der Schaftebene des Femurteils und der Vertikalebene 16 bis 21 mm beträgt, daß der Schaftabstand zwischen der Schaftebene des Tibiateils und der Schaftebene des Femurteils mit 0,5 bis 4 mm klein ist verglichen mit den Horizontalabständen zwischen der Vertikalebene und den jeweiligen Schaftebenen, daß der Vertikalabstand 20 bis 24 mm beträgt, und daß bei gleichmäßiger Belastung der beiden Teile die am Femurteil befestigten bogenförmigen Flügel auf der Auflagefläche gleiten, die bei einseitiger Belastung eines der beiden Flügel auf ihrer dem belasteten Flügel gegenüberliegenden Fläche auf Druck belastet sind.

Diese Kniegelenkendoprothese erfüllt drei Voraussetzungen, die sehr wesentlich für eine Anpassung der mit der Kniegelenkendoprothese durchführbaren Schwenkbewegungen an die Schwenkbewegungen natürlicher Kniegelenke sind:
1. Quer zur Beugeebene verläuft die sich durch den Stiel des Femurteils erstreckende Längsachse um einen kleinen Betrag versetzt gegenüber der Längsachse, die sich durch den Stiel des Tibiateils erstreckt. Die Versetzung erfolgt in der Weise, daß der Schaft des Tibiateils einen kleineren Abstand vom hinteren Teil der Prothese einhält als der Schaft des Femurteils. Durch diese Anordnung der Längsachsen werden einerseits die die Kräfte vom Femurteil in den Tibiateils und umgekehrt problemlos in den jeweils anderen Teil übergeleitet. Auf diese Weise erfolgt eine optimale Zuordnung und Führung des Femurs gegenüber dem Tibia.
2. Bezüglich der auf diese Weise definierten Schaftebenen ist die Schwenkachse des Scharniergelenkes in den hinteren Teil der Prothese verlagert. Dabei werden genaue Horizontalabstände eingehalten, die je nach den bei einem Patienten vorgefundenen Verhältnissen in gewissen Grenzen variiert werden können. Auf diese Weise ist einerseits dafür gesorgt, daß der Kraftfluß in optimaler Weise vom Femurteil bzw. Tibiateil in den jeweils anderen Teil erfolgt und in die zugeordneten Knochen eingeleitet wird. Durch die Zuordnung des Tibiateils zum Femurteil erfolgt bei richtiger Implantation der Prothese eine günstige Belastung der Knochen. Andererseits werden im hinteren Teil der Prothese Verhältnisse geschaffen, die eine problemlose Unterbringung der Weichteilummantelung auch im Bereich einer dem hinteren Teil der Prothese benachbarten Beuge ermöglicht. Einklemmungen oder Quetschungen dieser Ummantelungen sind auch in der Beuge nicht möglich. Die beiden Prothesenteile können resektionssparend in die jeweiligen Knochen eingesetzt werden. Die implantierte Prothese wird der anatomischen Zuordnung des Femurs zum Tibia gerecht. Schließlich bewirkten die Anordnung der Schäfte in zwei nur unwesentlich gegeneinander versetzten planparallelen Ebenen, daß die Kräfte in einer Belastungsachse verlaufen, die sich vor der Scharnierachse erstreckt. Durch diese Anordnung der Belastungsachse wird eine hohe Stabilität der Endoprothese in Streckstellung erzeugt. Durch diese dem System immanente Stabilisierung ergibt sich für den Träger der Endoprothese eine hohe Standfestigkeit in Streckstellung.
3. Darüber hinaus hält die Schwenkachse einen Vertikalabstand zu einer Verschiebeebene ein, auf der sich der dem Tibiateil benachbarte Teil des Femurteils verschiebt, wenn mit Hilfe der Kniegelenkendoprothese Schwenkbewegungen ausgeführt werden. Auf diese Weise wird eindeutig festgelegt, daß die Verschiebeebene in Richtung auf den Tibiateil unterhalb der Schwenkachse des Scharniergelenkes verläuft. Dadurch findet eine optimale Aufnahme der Kräfte statt, die vom Femurteil in den Tibiateil übergeleitet werden. Eine zusätzliche Absicherung der Kraftüberleitung über am Femurteil befestigte Kufen ist möglich, ohne daß diese die notwendigen Schwenkbewegungen beeinträchtigen. Diese Kufen können insbesondere kritische Querkräfte aufnehmen, die dazu führen könnten, daß der Femurteil gegenüber dem Tibiateil quer zur Beugeebene verlaufende Nickbewegungen ausführt. Gerade diese Nickbewegungen führen zu hohen Belastungen im Gelenk, die einen Materialflow im Achslager erzeugen können. Durch diesen Materialflow wird das im Achslager verwendete Material weich, so daß das Achslager zerstört wird. Da die Schwenkachse oberhalb der Verschiebeebene verläuft, findet eine optimale Abstützung des Femurteils auf der Verschiebeebene statt, die auf dem Tibiateil ausgebildet ist. Das Auftreten von Nichtbewegungen ist daher weitgehend ausgeschlossen, obgleich angesichts der Notwendigkeit einer resektionssparenden Unterbringung der gesamten Kniegelenkendoprothese das Scharniergelenk nur eine relativ kurze Mittelachse aufweist.

Die Kniegelenkendoprothese ist auf Grund der drei geschilderten Parameter so ausgebildet, daß sie die wesentlichen natürlichen Bewegungen, die in einem Kniegelenk ablaufen, weitgehend simulieren kann. Auf diese Weise ist dafür gesorgt, daß sowohl der Femurteil als auch der Tibiateil auf Dauer fest in den ihnen jeweils benachbarten Knochen befestigt sind und sich nicht leicht lockern können. Durch die problemlose Einleitung der Kräfte in die Knochen ist Beanspruchung der jeweiligen Knochen gering.

Bei einem Abstand von 0,5 mm bis 4 mm zwischen den beiden Schaftebenen wird einerseits die Verschwenkbarkeit des Tibiateils gegenüber dem Femurteil günstig beeinflußt, zum anderen verlaufen die Belastungen in Streckstellung der Endoprothese im wesentlichen in einer Belastungsebene, die zu einer günstigen Überleitung der Kräfte führt und eine besonders große Stabilität in Streckstellung der Endoprothese aufweist. Diese Vorteile treten besonders günstig hervor, wenn der Schaftabstand zwischen 1,5 und 2,5 mm beträgt.

Bei einem Horizontalabstand im Bereich des Oberschenkels von etwa 16 bis 21 mm findet eine optimale Umlenkung der Kräfte statt, die vom Femurteil in den Tibiateil und umgekehrt in beliebigen Schwenkstellungen des Endogelenkes eingeleitet werden. Dieser Horizontalabstand ermöglicht auch die Ausbildung einer Schwenkachse des Scharniergelenkes in eine Lage, in der Schwenkbewegungen um diese Schwenkachse stattfinden können, ohne daß die in der Beuge des Kniegelenks vorhandenen Weichteile eingeklemmt werden. Dieser Horizontalabstand hat sich für einen sehr großen Durchschnitt aller zu implantierenden Kniegelenke als optimal erwiesen. Bei einer großen Anzahl von Patienten treten bei Einhaltung dieses Horizontalabstandes optimale Beugeverhältnisse auf.

Bei einem Vertikalabstand von 20 bis 24 mm können optimale Anpassungen an die beim jeweiligen Patienten vorgefundene Anatomie vorgenommen werden, so daß die notwendigen Knochenresektionen sehr sparsam durchgeführt werden können. Darüber hinaus ermöglicht dieser Vertikalabstand die Ausbildung einer die Kräfte ohne Schwierigkeiten übertragenden Verschiebeebene. Bei diesem Vertikalabstand können sowohl der Femurteil als auch der Tibiateil so gestaltet werden, daß die beiden Teile in ihrer Gestaltung gut im jeweiligen Knochen verankert werden können, wobei sparsame Resektionen möglich sind. Ein im Femurteil vorgesehenes Gehäuse besitzt Wandungen von relativ kleiner Wandstärke, die geeignet sind, die bei diesem Vertikalabstand auftretenden Biegekräfte aufzunehnmen. In ähnlicher Weise ist auch ein in das Gehäuse hineinragender Steg des Tibiateils von einer relativ kleinen Materialstärke, die aber angesichts des gewählten Vertikalabstandes ausreicht, um die auftretenden Biegemomente übertragen zu können, ohne daß deswegen in diesem Teil bleibende Verformungen auftreten.

Gemäß bevorzugten Ausführungsformen der Erfindung beträgt der Horizontalabstand etwa 16 mm und der Vertikalabstand etwa 23 mm. Dadurch wird ein guter Kompromiß zwischen der erwünschten Gewebeentlastung und der gleichzeitig erforderlichen Erhaltung der Knochenstabilität geschlossen. Größere Horizontalabstände führen zu schlechten Implantationsbedingungen und hohen Belastungen im Kniescheibengelenk. Bei einer Verkleinerung des Horizontalabstandes ist eine größere Knochenresektion zum Zwecke der Verankerung der Endoprothese erforderlich. Auf Grund der erfindungsgemäßen Anordnung der Schwenkachse kann jedoch sowohl eine Gewebeentlastung als auch eine Erhaltung der Knochenfestigkeit gewährleistet werden. Die für den femuralen und tibialen Teil gewählten jeweiligen Horizontalabstände führen im Vergleich zu anderen Sytemen zu einer wesentlichen Druckentlastung des Kniescheibengelenkes, weil kein Vorschub im Bereich der Oberschenkelrolle auftreten kann. Dieser Vorschub im Bereich der Oberschenkelrolle stellt eine vollkommen unphysiologische Belastung des Endoprothesengelenkes dar. Diese Druckentlastung des Kniescheibengelenkes ist besonders wichtig, weil das Kniescheibengelenk in seiner natürlichen Funktion ohne Gelenkersatz üblicherweise erhalten wird. Durch Beibehaltung des natürlichen Kniescheibengelenkes entsteht eine beachtliche Resektionsersparnis, so daß nur wahlweise in speziellen Situationen das Kniegelenk ersetzt wird, überwiegend bei Korrekturoperationen.

Gemäß einer weiteren bevorzugten Ausführungsform der Erfindung weist das Gehäuse auf seinen dem Gehäuseinnenraum abgewandten Außenwandungen seiner seitlichen Begrenzungen je einen Flügel auf, von denen jeder in eine vom Gehäuseinnenraum abgewandte Richtung weist und sich bogenförmig an einem unteren Rand des Gehäuses erstreckt, der dem Tibiateil zugewandt ist, in Richtung auf den die Flügel eine Gleitfläche aufweisen.

Weitere Einzelheiten und Merkmale der Erfindung ergeben sich aus der nachfolgenden ausführlichen Beschreibung und den beigefügten Zeichnungen, in denen bevorzugte Ausführungsformen der Erfindung beispielsweise veranschaulicht sind.

In den Zeichnungen zeigen:

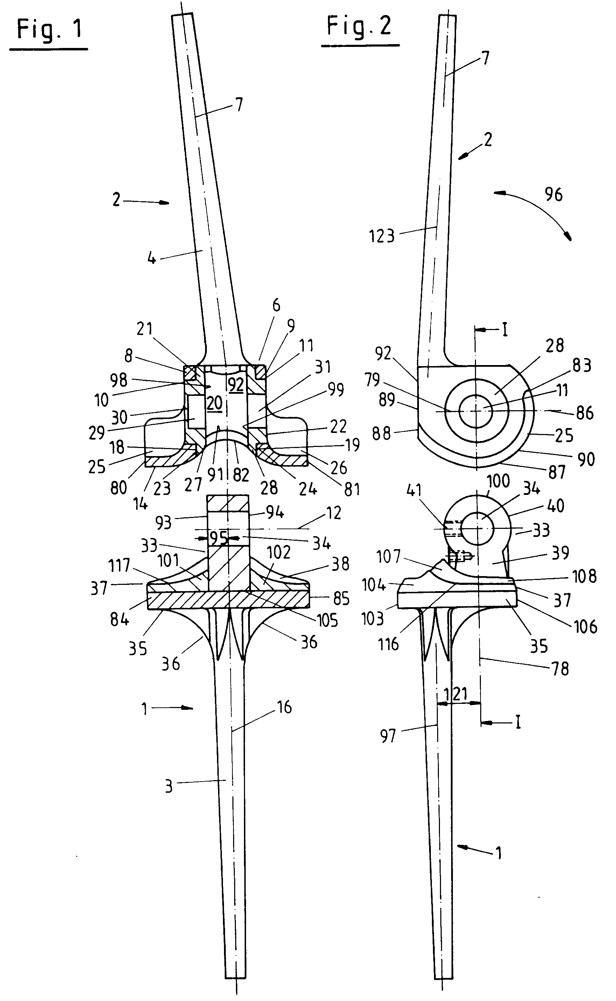

- Fig. 1:: Einen Längsschnitt durch eine Endoprothese mit vom Tibiateil abgenommenen Femurteil gem. Schnittlinie I-I in Fig. 2,
- Fig. 2:: eine Seitenansicht der Endoprothese gem. Fig. 1,

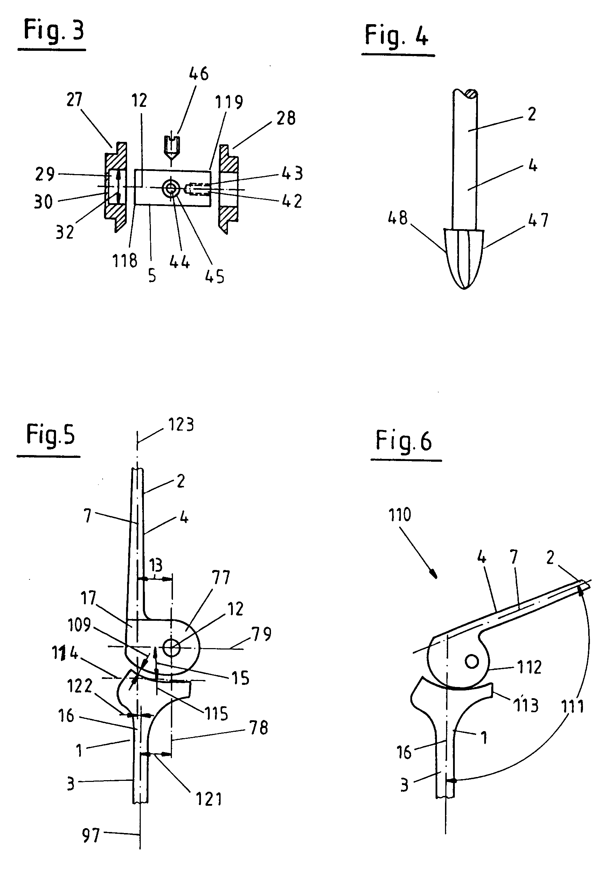

- Fig. 3:: einen Längsschnitt durch Zwei zur Aufnahme der Welle vorgesehene Lagerschalen und eine Ansicht einer Welle mit einer Zentrierschraube,
- Fig. 4:: eine teilweise Darstellung eines Prothesenschaftes mit Zentrierstern,
- Fig. 5:: eine skizzenhafte Darstellung der gestreckten Endoprothese mit Achsenlagen in Relation zu den Schäften und zur Abrollfläche,
- Fig. 6:: eine skizzenhafte Darstellung der verschwenkten Endoprothese,

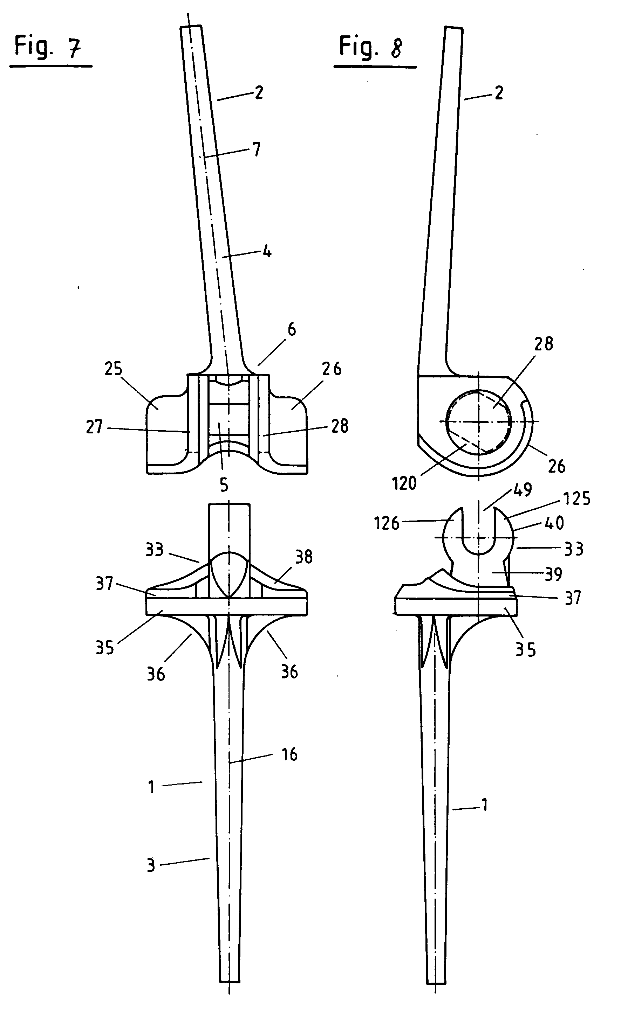

- Fig. 7:: eine Vorderansicht einer Kniegelenkendoprothese mit zusammensteckbarem Tibia- und Femurteil,
- Fig. 8:: eine Seitenansicht der Prothese nach Fig. 7,

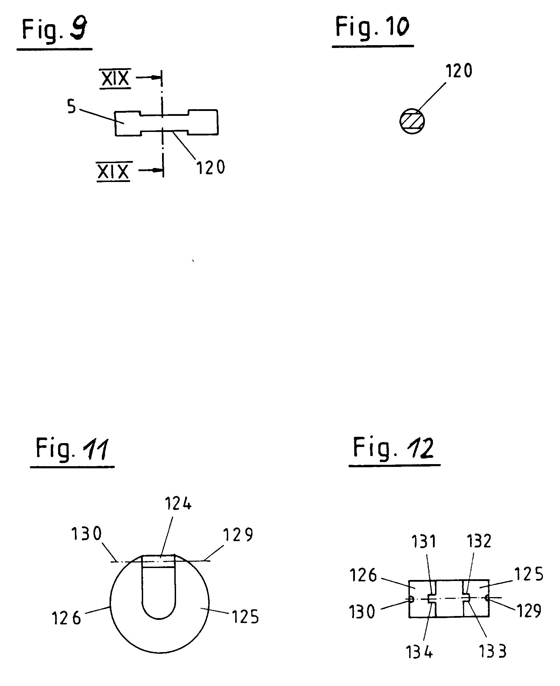

- Fig. 9:: eine Seitenansicht einer Welle,
- Fig.10:: einen Querschnitt durch eine Welle entlang der Schnittlinie XIX-XIX in Fig. 9,
- Fig.11:: eine Seitenansicht eines anderen Führungselementes und
- Fig.12:: eine Draufsicht auf ein anders Führungselement.

Eine Kniegelenkendoprothese besteht im wesentlichen aus einem Tibiateil (1) und einem Femurteil (2). Das Tibiateil (1) weist einen Tibiaschaft (3), das Femurteil (2) einen Femurschaft (4) auf. Die beiden Schäfte (3, 4) weisen in einander abgewandte Richtungen und dienen zur Befestigung der Teile (1, 2) in einem ihnen jeweils benachbarten Knochen.

Im Bereich der dem Tibiateil (1) zugewandten Ausdehnung des Femurteils (2) ist ein eine Welle (5) führendes Gehäuse (6) angeordnet. Diese Welle ist im Tibiateil (1) befestigt und im Femurteil (2) drehbar gelagert. Bei der Ausführung von Drehbewegungen der Welle (5) führen die beiden Teile (1, 2) Schwenkbewegungen in einer Beugeebene (96) gegeneinander aus. Diese Beugebewegungen führen von einer gestreckten Stellung, in der sich durch jeden der beiden Schäfte (3, 4) eine senkrecht zur Beugeebene (96) verlaufende Schaftebene (97, 123) erstreckt, in eine verschwenkte Stellung (110), in der eine sich durch den Femurschaft (4) erstreckende Femurlängsachse (7) mit einer sich durch den Tibiaschaft (3) erstreckenden Tibialängsachse (16) einen Beugewinkel (111) zwischen sich einschließt. Dieser Beugewinkel (111) umschließt einen hinteren Teil (112) des Femurteils (2) und einen hinteren Teil (113) des Tibiateils (1).

Im Bereich seiner sich im wesentlichen planparallel zur Beugeebene (96) erstreckenden seitlichen Begrenzungen (8, 9) weist das Gehäuse Ausnehmungen (10, 11) auf. Diese Ausnehmungen (10, 11) sind im wesentlichen zentrisch zu einer sich durch die Welle (5) erstreckenden Schwenkachse (12) ausgerichtet, die sich im wesentlichen quer zur Beugeebene (96) erstreckt. Durch die Schwenkachse (12) erstreckt sich eine Vertikalebene (78), die planparallel in einem Horizontalabstand (13) zu der sich durch den Femurschaft (4) erstreckenden Schaftebene (123) und in einem Horizontalabstand (121) zu der sich durch den Tibiaschaft (3) erstreckenden Schaftebene (97) verläuft. Darüber hinaus erstreckt sich die Schwenkachse (12) durch eine Horizontalebene (79), die in Richtung auf den Tibiateil (1) einen Vertikalabstand (15) zu einer horizontalen Verschiebeebene (114) besitzt. Diese horizontale Verschiebeebene (114) verläuft tangential an eine Linie (115), die sich senkrecht zur Beugeebene (96) durch eine dem Femurteil (2) gegenüber auf dem Tibiateil (1) ausgebildete Auflagefläche (117) erstreckt. Diese Auflagefläche (117) ist in Richtung auf den Femurteil (2) gegenüber einer Gleitfläche (14) ausgebildet, die geeignet ist, auf der Auflagefläche (117) gleitend Bewegungen auszuführen, wenn das Tibiateil (1) um die Schwenkachse (12) Schwenkbewegungen bezüglich des Femurteils (2) ausführt.

In der quer zur Beugeebene (96) verlaufenden Schaftebene (97) verläuft der Tibiaschaft (3) in Richtung einer Tibialängsachse (16) und der Femurschaft (4) in Richtung einer Femurlängsachse (7). Demgegenüber besitzt der Femurschaft (4) in einer quer zur Schaftebene (123) verlaufenden Vorderansicht eine Schrägstellung gegenüber dem Tibiaschaft (3). Diese Schrägstellung entspricht einem mittleren Valguswinkel.

Die Ausnehmungen (10, 11) dienen zur Aufnahme von Lagerschalen (27, 28), in denen die Welle (5) gelagert ist. Diese Lagerschalen (27, 28) besitzen jeweils einen Bund (23, 24), mit dem sie in die Ausnehmungen (10, 11) hineinragen. An diesen Bund (23, 24) schließen sich Auflageflächen (21, 22) an, mit denen die Lagerschalen (27, 28) in einen vom Gehäuse (6) umschlossenen Gehäuseinnenraum (20) hineinragen. Diese Auflagenflächen (21, 22) liegen mit ihren dem Gehäuseinnenraum (20) abgewandten Außenflächen (18, 19) auf den seitlichen Begrenzungen (8, 9) des Gehäuses (6) auf. Auf diese Weise wird der Gehäuseinnenraum (20) beidseits von Innenwandungen (98, 99) begrenzt, die von den Lagerschalen (27, 28) gebildet werden. Diese Lagerschalen (27, 28) bestehen aus Polyäthylen und stellen daher einen idealen Gleitpartner für einen Steg (33) dar, mit dem der Tibiateil (1) in den Gehäuseinnenraum (20) hineinragt. Dabei sind die Abmessungen des Steges (33) so gewählt, daß dieser mit seinen Seitenwandungen (93, 94) auf den Innenwandungen (98, 99) geführt ist.

Während eine der beiden Lagerschalen (27, 28) mit einem Durchgangsloch (31) versehen ist, in dem die Welle (5) gelagert ist, ist in der anderen Lagerschale (27) lediglich ein Sackloch (29) vorgesehen, in dem die Welle (5) gelagert ist. Dieses Sackloch (29) wird von einem es abschließenden Boden (30) begrenzt, der die Welle (5) in Längsrichtung der Schwenkachse (12) führt. Sowohl das Durchgangsloch (31) als auch das Sackloch (29) besitzen einen Innendurchmesser (32), der einem äußeren Durchmesser der Welle (5) entspricht, so daß die Welle (5) sowohl im Durchgangsloch (31) als auch im Sackloch (29) geführt ist.

Die das Gehäuse (6) begrenzenden seitlichen Begrenzungen (8, 9) tragen auf ihren dem Gehäuseinnenraum (20) abgewandten Außenflächen Flügel (25, 26), die sich vom Gehäuse (6) in eine vom Innenraum (20) abgewandte Richtung erstrecken. Diese Flügel (25, 26) erstrecken sich bogenförmig an einer Unterkante (82) in jeweils voneinander wegweisende Richtungen. Diese Unterkante (82) ist dem Tibiateil (1) zugewandt. Die Gleitfläche (14) befindet sich auf einer dem Tibiateil (1) zugewandten Unterfläche der Flügel (25, 26).

Die Flügel (25, 26) besitzen parallel zur Richtung der Schwenkachse (12) eine relativ große Breite, so daß sie eine breite Gleitfläche (14) aufspannen, mit der sie auf einer Lagerfläche (38) geführt sind, die auf dem Tibiateil (1) angeordnet ist. Diese Lagerfläche (38) stellt eine gewölbte Auflagefläche (117) dar, deren Wölbung im wesentlichen derjenigen der Flügel (25, 26) nachgebildet ist. Die Breite jedes der beiden Flügel (25, 26) entspricht etwa der halben Breite, die das Gehäuse (6) aufweist. Die Gleitflächen (14) der beiden Flügel (25, 26) weisen jeweils eine in Richtung auf den Tibiateil (1) ballig gewölbte schwache Neigung auf, die sich vom Gehäuse (6) in Richtung auf Außenkanten (80, 81) erstreckt. Diese Außenkanten (80, 81) begrenzen die Flügel (25, 26) auf ihrer dem Gehäuse (6) gegenüberliegenden Seite. Diese Außenkanten (80, 81) kongruieren im wesentlichen mit Außenkanten (84, 85) einer Auflageplatte (35), die den Tibiateil (1) gegenüber dem Femurteil (2) begrenzt. Diese Auflageplatte (35) spannt eine quer zur Richtung der Schaftebene (97) verlaufende Auflagefläche (117) auf.

Die Flügel (25, 26) erstrecken sich über einen in etwa zur Schwenkachse (12) konzentrisch verlaufenden Bogen von etwa 180 ° entlang der Unterkante (82) des Femurteils (2). Jeder Flügel (25, 26) weist ein in Richtung auf den hinteren Teil (112) des Femurteils weisendes Ende (83) auf, das von einer von der Horizontalebene (79) mit der Unterkante (82) des Gehäuses (6) gebildeten Schnittlinie (86) kreisbogenförmig konzentrisch zur Mittelachse (12) verläuft und im gestreckten Zustand der Kniegelenkendoprothese ein knapp oberhalb der Horizontalebene (79) liegendes hinteres Ende (83) aufweist. Dieses hintere Ende (83) ist der Auflagefläche (117) benachbart, wenn der Femurteil (2) gegenüber dem Tibiateil (1) verschwenkt ist.

Jeder Flügel (25, 26) erstreckt sich entlang einem Bogen (87) vom hinteren Ende (83) zu einem diesen gegenüberliegenden vorderen Ende (88), das in einer dem hinteren Teil (112) des Femurteils (2) gegenüberliegenden vorderen Abschlußfläche (89) des Gehäuses (6) liegt. Das vordere Ende (88) liegt im gestreckten Zustand der Prothese um etwa eine gleiche Entfernung in Richtung auf den Tibiateil (1) unterhalb der Horizontalebene (79) wie das hintere Ende (83) oberhalb dieser Horizontalebene (79) liegt.

Der Bogen (87) weist vom hinteren Ende (83) zum vorderen Ende (88) verschiedene Krümmungsradien auf. So ist er im Bereich seines hinteren Endes (83) und im Bereich seines vorderen Endes (88) schwächer gekrümmt als in seinem Mittelbereich (90). Die vorderen Enden (88) der beiden Flügel (25, 26) sind über einen kurzen vom Tibiateil (1) abgewandt gewölbten kurzen Bogen (91) miteinander verbunden, der in einer das Gehäuse (6) begrenzenden vorderen Stirnwand (92) ausgebildet ist.

Der Steg (33) weist eine Ausnehmung (34) auf, die als eine im wesentlichen zentrisch zur Schwenkachse (12) verlaufende Bohrung ausgebildet ist. Diese dient zur Aufnahme der Welle (5). Der Steg (33) erhebt sich auf der Auflageplatte (35), zu deren horizontaler Fläche die Seitenwandungen (93, 94) senkrecht verlaufen. Diese Seitenwandungen (93, 94) weisen einen etwa gleichgroßen Abstand (95) von der sich mittig durch den Tibiateil (1) erstreckenden Beugeebene (96) auf. Damit verlaufen die Seitenwandungen (93, 94) auch planparallel zu den Innenwandungen (98, 99) des Gehäuses (6), auf denen sie geführt sind.

Der Gehäuseinnenraum (20) besitzt einen quer zur Beugeebene (96) im wesentlichen rechteckigen Querschnitt. Der Gehäuseinnenraum (20) ist in Richtung auf das vordere Ende (88) von der im wesentlichen planparallel zur Schaftebene (123) verlaufenden Abschlußfläche (89) begrenzt, während er in Richtung auf das hintere Ende (83) offen ist.

Der Steg (33) erhebt sich mittig auf der Auflageplatte (35). Er ist als eine Öse ausgebildet, deren dem Femurteil (2) zugewandte obere Begrenzung (100) als eine konzentrisch zur Ausnehmung (34) verlaufende Rundung ausgebildet ist. Der Steg (33) erstreckt sich in Richtung auf den Femurteil (2) durch eine Lagerschale (37), die auf der Auflageplatte (35) angeordnet ist. Diese Auflageplatte (35) ist mit Hilfe von Rippen (36) gegenüber dem Tibiaschaft (3) abgestützt.

Die Lagerschale (37) umgreift den Steg (33) U-förmig und erstreckt sich entlang den Seitenwandungen (93, 94) mit zwei Stegen (101, 102), die in einem der vorderen Abschlußfläche (89) zugewandten vorderen Teil (103) der Auflageplatte (35) durch ein Joch (104) miteinander verbunden sind. Zwischen den beiden Stegen (101, 102) erstreckt sich eine Öffnung (105) bis zu einem dem vorderen Teil (103) gegenüberliegenden hinteren Teil (106) der Auflageplatte (35). Auf diesem hinteren Teil (106) der Auflageplatte erhebt sich durch die Öffnung (105) der Steg (33).

Auf der Lagerschale (37) ist die Lagerfläche (38) ausgebildet, die der Gleitfläche (14) zugewandt ist. Die Lagerfläche (38) verläuft auf einer dem Femurteil (2) zugewandten Oberfläche der Stege (101, 102) vom vorderen Teil (103) in Richtung auf den hinteren Teil (106), in den sie einmünden.

Die Lagerfläche (38) weist einen dem Joch (104) zugewandten vorderen Teil (107) auf, der dem vorderen Ende (88) der Flügel (25, 26) gegenüberliegt. Gegenüber einem in den hinteren Teil der Lagerschale (37) einmündenden flachen Teil (108) der Lagerfläche (38) ist der vordere Teil (107) in Richtung auf den Femurteil (2) angehoben. Der flache Teil (108) der Lagerfläche (38) erstreckt sich innerhalb der Auflagefläche (117). Zwischen der Lagerfläche (38) und der ihr zugewandten Gleitfläche (14) der Flügel (25, 26) besteht im gleichmäßig belasteten Zustand der Prothese ein Gleitspiel. Bei einseitigen Belastungen der Prothese stützt sich die Gleitfläche (14) des belasteten Flügels (25, 26) auf der Lagerfläche (38) ab und verhindert auf diese Weise, daß sich der Steg (33) im Gehäuse (6) bei der Durchführung von Schwenkbewegungen verkantet.

Die Lagerfläche (38) weist in Längsrichtung von ihrem flachen Teil (108) zu ihrem vorderen Teil (107) eine Wölbung (116) auf, die in ihrem dem vorderen Teil (107) zugewandten Drittel einen relativ steilen Anstieg aufweist. Darüber hinaus besitzt die Lagerfläche (38) in ihrer quer zur Längsrichtung verlaufenden Querrichtung eine Wölbung, die der Wölbung der Gleitfläche (14) angepaßt ist. Auf dieser in Querrichtung verlaufenden Wölbung werden die Flügel (25, 26) beim Ausführen von Beugebewegungen geführt. Diese in Querrichtung verlaufende Wölbung stabilisiert das Kniegelenk in gestreckter Stellung dadurch, daß der Tibiateil (2) im vorderen Teil (103) der Auflageplatte (35) die Unterkante (82) des Gehäuses (6) formschlüssig führt.

Zweckmäßigerweise ist die Lagerschale (37) aus Polyäthylen ausgebildet, so daß sie einen idealen Gleitpartner zu den Gleitflächen (14) der Flügel (25, 26) ausbildet. Auf diese Weise ist auch bei heftiger Belastung der beiden Teile (1, 2) damit zu rechnen, daß die Gleitfläche (14) Gleitbewegungen auf der Lagerschale (27, 28) ausführen kann.

Die Welle (5) ist in der Ausnehmung (34) mit Hilfe einer Madenschraube (46) befestigt. Diese erstreckt sich durch eine Bohrung (41), die quer zur Schwenkachse (12) durch den Steg (33) in Richtung auf die Ausnehmung (34) verläuft. Der Bohrung (41) entspricht eine in der Welle (5) vorgesehene Bohrung (44), in die die Madenschraube (46) hineinragt, wenn sie in die Bohrung (41) hineingeschraubt ist. Auf diese Weise wird die Welle (5) innerhalb der Ausnehmung (34) fixiert. Die Madenschraube (45) wird in die Bohrung (41) so weit hineingeschraubt, bis ihr der Welle (5) abgewandtes Ende unterhalb einer Oberfläche des Steges (33) innerhalb der Bohrung (41) liegt. Innerhalb der Bohrung (41) wird die Madenschraube (45) mit Knochenzement fixiert und auf diese Weise vermieden, daß sie sich aus der Bohrung (41) während des Gebrauchs der Prothese herausdreht und mit ihrem aus der Bohrung (41) herausragenden Ende das Gehäuse (6) zerstört und mit ihrem anderen Ende die Verbindung mit der WWelle (5) verliert.

Nachdem diese Fixierung vorgenommen worden ist, ragt die Welle (5) mit ihren beiden Enden (118, 119) beidseits aus der Ausnehmung (34) heraus, so daß diese Enden (118, 119) in den Löchern (29, 31) der Lagerschalen (27, 28) gelagert werden können.

Darüber hinaus kann in der Welle (5) eine Bohrung (42) angeordnet sein, die sich in Richtung der Schwenkachse (12) erstreckt und mit einem Innengewinde (43) versehen ist. Diese Bohrung (42) dient der Montage der Welle (5), wenn diese durch das Durchgangsloch (31) hindurch in das Sackloch (29) eingepaßt wird und anschließend so ausgerichtet wird, daß die Madenschraube (46) durch die Bohrung (41) hindurch in die mit einem Innengewinde (45) versehene Bohrung (44) eingeschraubt wird.

Im Bereich der einander abgewandten Enden der Schäfte (3, 4) können Zentriersterne (47) angeordnet sein. Es ist aber auch möglich, nur im Bereich eines der Schäfte einen Zentrierstern (47) vorzusehen oder die Endoprothese ohne Zentriersterne (47) zu implantieren. Der Zentrierstern (47) ist im wesentlichen zentrisch zur Schaftachse (17) ausgebildet und verjüngt sich in eine dem Schaft (3, 4) abgewandte Richtung. Der Zentrierstern (47) weist ihn stabilisierende und beim Einführen in eine Knochenausnehmung zentrierende Flügel (48) auf.

Gemäß einer anderen Ausführungsform der Endoprothese ist die Welle (5) fest im Bereich des Femurteils (2) gelagert und die Ausnehmung (34) im Steg (33) mit einer in Richtung des Femurteils (2) orientierten Einführöffnung (49) versehen. Die Einführöffnung (49) ermöglicht ein Zusammenfügen des Tibiateils (1) und des Femurteils (2) ohne seitliches Einführen des Lagerzapfens (5). Die Einbringung einer zusätzlichen Bohrung in den Femurknochen kann hierdurch vermieden werden. Dabei ist die Einführöffnung (49) so ausgebildet, daß die in den seitlichen Begrenzungen (8, 9) befestigte Welle (5) mit einer Schmalstelle (120) in die Einführöffnung (49) eingeführt werden kann. Diese Schmalstelle (120) ist bei gestreckter Stellung des Kniegelenks gegenüber der Einführöffnung (49) verschwenkt. Zusätzlich ist aber auch noch die Möglichkeit gegeben, die Einführöffnung (49) durch eine sich quer zu ihr erstreckende Schraube zu verschließen, die als Madenschraube ausgebildet sein sollen. Es ist jedoch auch möglich, die Einführöffnung (49) mit einem Riegel (124) zu verschließen, der sich zwischen zwei den Steg (33) ausbildenden Schenkeln (125, 126) an deren dem Femurteil (2) zugewandten Enden (127, 128) erstreckt. Dieser Riegel (124) wird zweckmäßigerweise mit einer Madenschraube (129) an den Schenkeln (125, 126) befestigt. Es ist jedoch zur besseren Führung des Riegels (124) auch möglich, diesen mit zwei Federn (131, 132) in entsprechenden Nuten (133, 134) der Schenkel (125, 126) zu führen. Um diesen Verschluß in die Einführöffnung (49) leicht einsetzen zu können, wird der Steg (33) so mit der Einführöffnung (49) versehen, daß diese im verschwenkten Zustand des Kniegelenkes innerhalb des Gehäuses (6) leicht zugänglich ist. Zu diesem Zwecke verläuft die Einführöffnung (49) im gestreckten Zustand der Endoprothese schräg in eine dem hinteren Teil (83) abgewandte Richtung vorne durch den Steg ((33).

Die Lagerschalen (27, 28, 37) sind vorzugsweise aus einem dauerbeanspruchbaren Kunststoff, beispielsweise Polyäthylen, ausgebildet. Die Welle (5) besteht aus einem Metall. Es ist aber auch möglich, die Welle aus einem Metallkern auszubilden, der von einer beispielsweise aus Polyäthylen ausgebildeten Hülle umschlossen ist. In diesem Falle bestehen die Lagerschalen aus Metall. Die die Lagerschale (37) beaufschlagenden Flügel (25, 26) sind vorzugsweise aus einem harten Metall ausgebildet und ihre Gleitflächen (14) sind poliert.

Für den Horizontalabstand (13) ist eine Bemaßung von etwa 16 bis 21 mm vorgesehen. Besonders vorteilhaft in Bezug auf eine Vielzahl von Anwendungen ist ein Horizontalabstand von 16 mm. Der Vertikalabstand (15) wird in einem Bereich von 20 bis 24 mm realisiert. Insbesondere eine Bemaßung im Bereich von 22 mm, führt zu einer universellen Verwendbarkeit der Endoprothese.

Die Schäfte (3, 4) sind gleichfalls aus Metall ausgebildet und weisen in Relation zu ihren Durchmessern eine große Längserstrekkung in Richtung der Schaftachse (4, 16) auf.

In Richtung auf ihre einander abgewandten Enden verjüngen sich die Schäfte (3, 4). Zur Erleichterung von Wechseloperationen sind die Schäfte (3, 4) vorzugsweise hinterschneidungsfrei ausgebildet. Durch eine biegeelastische Ausbildung der Schäfte (3, 4) können diese dem Biegeverhalten des jeweiligen Knochens angepaßt werden, so daß dadurch die Gefahr einer Lockerung der Schäfte (3, 4) gemindert wird. Darüber hinaus besitzen die Schäfte (3, 4) einen rechteckigen Querschnitt mit abgerundeten Kanten. Diese Querschnittsform hat sich als besonders günstig erwiesen, um einerseits die auftretenden Kräfte aufnehmen zu können, andererseits aber auch, um zu verhindern, daß sich die Schäfte (3, 4) unter der Belastung von Verdrehkräften im Knochen lockern. Schließlich bietet diese Querschnittsform eine hohe Sicherheit gegen möglicherweise auftretende Kerbwirkungen.

## Patentansprüche

1. Kniegelenkendoprothese mit einem Femurteil (2) und einem Tibiateil (1), die um ein sie miteinander verbindendes Scharniergelenk, dessen Schwenkachse (12) einen nicht veränderbaren Abstand sowohl vom Femurteil (2) als auch vom Tibiateil (1) besitzt, von einer gestreckten Stellung in eine abgewinkelte Stellung in einer Beugeebene (96) verschwenkbar gelagert sind und jeweils Schäfte (3, 4) aufweisen, die in abgewinkelter Stellung einen Beugewinkel (11) zwischen sich einschließen und in gestreckter Stellung in einander entgegengesetzte Richtungen weisen, wobei das Femurteil (2) an ihm befestigte bogenförmige Flügel (25, 26) aufweist und sich durch die Schäfte (3, 4) jeweils eine Längsachse (7, 16) erstreckt, die in gestreckter Stellung im wesentlichen in quer zur Beugeebene (96) verlaufenden einander planparallelen Schaftebenen (97, 123) angeordnet sind, wobei von den Schaftebenen (97, 123) die des Femurteils (2) einen größeren Horizontalabstand (13) von einer planparallel zu ihnen durch die Schwenkachse (12) verlaufende Vertikalebene (78) in Richtung auf einen vom Beugewinkel (111) eingeschlossenen hinteren Teil (112) des Femurteils (2) einhält als die Schaftebene (97) des Tibiateils (1)und wobei zwischen der Schaftebene (97) des Tibiateils (1) und der Schaftebene (123) des Femurteils (2) ein Schaftabstand (122) besteht und eine senkrecht zur Vertikalebene (78) durch die Schwenkachse (12) verlaufende Horizontalebene (79) in Richtung auf den Tibiateil (1) einen Vertikalabstand (15) zu einer horizontalen Verschiebeebene (114), die sich tangential zu einer auf dem Tibiateil (1) ausgebildeten Auflagefläche (117) erstreckt, einhält, dadurch gekennzeichnet, daß der Horizontalabstand (13) zwischen der Schaftebene (123) des Femurteils (2) und der Vertikalebene (78) 16 bis 21 mm beträgt, daß der Schaftabstand (122) zwischen der Schaftebene (97) des Tibiateils (1) und der Schaftebene (123) des Femurteiles (2) mit 0,5 bis 4 mm klein ist verglichen mit den Horizontalabständen (13, 121) zwischen der Vertikalebene (78) und den jeweiligen Schaftebenen (97, 123), daß der Vertikalabstand (15) 20 bis 24 mm beträgt und daß bei gleichmäßiger Belastung der beiden Teile (1, 2) die am Femurteil (2) befestigten bogenförmigen Flügel (25, 26) auf der Auflagefläche (117) gleiten, die bei einseitiger Belastung eines der beiden Flügel (25, 26) auf ihrer dem belasteten Flügel (25, 26) gegenüberliegenden Fläche auf Druck belastet ist.

2. Kniegelenkendoprothese nach Anspruch 1, dadurch gekennzeichnet, daß die Schaftebene (123) des Femurteils (2) planparallel zur Schaftebene (97) des Tibiateils (1) durch den Schaft (3) des Tibiateils (1) verläuft.

3. Kniegelenkendoprothese nach Anspruch 1 oder 2, dadurch gekennzeichnet, daß der Schaftabstand (122) 1,5 bis 2,5 mm beträgt.

4. Kniegelenkendoprothese nach einem der Ansprüche 1 bis 3, dadurch gekennzeichnet, daß der Horizontalabstand (13) etwa 16 mm beträgt.

5. Kniegelenkendoprothese nach einem der Ansprüche 1 bis 4, dadurch gekennzeichnet, daß der Vertikalabstand (15) etwa 21 mm beträgt.

6. Kniegelenkendoprothese nach einem der Ansprüche 1 bis 5, dadurch gekennzeichnet, daß mindestens einer der Schäfte (3, 4) der Teile (1, 2) in Relation zu ihrem Durchmesser lang ausgebildet ist.

7. Kniegelenkendoprothese nach einem der Ansprüche 1 bis 6, dadurch gekennzeichnet, daß mindestens einer der Schäfte (3, 4) sich in Richtung des dem anderen Schaft (3, 4) abgewandten Endes verjüngend ausgebildet ist.

8. Kniegelenkendoprothese nach einem der Ansprüche 1 bis 7, dadurch gekennzeichnet, daß mindestens einer der Schäfte (3, 4) hinterschneidungsfrei ausgebildet ist.

9. Kniegelenkendoprothese nach einem der Ansprüche 1 bis 8, dadurch gekennzeichnet, daß mindestens einer der Schäfte (3, 4) im Bereich seines dem anderen Schaft abgewandten Endes mit einem Zentrierstern (47) versehen ist.

10. Kniegelenkendoprothese nach einem der Ansprüche 1 bis 9, dadurch gekennzeichnet, daß der Zentrierstern (47) aus einem Kunststoff ausgebildet ist.

11. Kniegelenkendoprothese nach einem der Ansprüche 1 bis 10, dadurch gekennzeichnet, daß der Schäfte (3, 4) einen viereckigen Querschnitt aufweist, dessen Kanten abgerundet sind.

12. Kniegelenkendoprothese nach einem der Ansprüche 1 bis 11, dadurch gekennzeichnet, daß im Bereich der dem Tibiateil (1) zugewandten Ausdehnung des Femuirteils (2) ein Gehäuse (6) angeordnet ist, das im wesentlichen aus zwei einander etwa planparallel verlaufenden seitlichen Begrenzungen (8, 9) besteht, die sich vom Schaft (4) des Femurteils (2) in Richtung auf den Tibiateil (1) erstrecken.

13. Kniegelenkendoprothese nach einem der Ansprüche 1 bis 12, dadurch gekennzeichnet, daß im Bereich der seitlichen Begrenzung (8, 9) des Gehäuses (6) eine sich quer zur Beugeebene (96) durch das Gehäuse (6) erstreckende Welle (5) führende Lagerschalen (27, 28) angeordnet sind, von denen jeweils eine in jeweils einer seitlichen Begrenzung (8, 9) gelagert ist.

14. Kniegelenkendoprothese nach einem der Ansprüche 1 bis 13, dadurch gekennzeichnet, daß eine der beiden Lagerschalen (27) mit einem die Welle (5) aufnehmenden Sackloch (29) versehen ist.

15. Kniegelenkendoprothese nach einem der Ansprüche 1 bis 14, dadurch gekennzeichnet, daß die andere Lagerschale (28) ein die Welle (5) führendes Durchgangsloch (31) aufweist, das mit dem Sackloch (29) fluchtet.

16. Kniegelenkendoprothese nach einem der Ansprüche 1 bis 15, dadurch gekennzeichnet, daß Ausnehmungen (10, 11) des Gehäuses (6) jeweils einen Bund (23, 24) der Lagerschalen (27, 28) aufnehmen, die mit jeweils einer Außenfläche (18, 19) auf den seitlichen Begrenzungen (8, 9) aufliegen und in Richtung eines von den seitlichen Begrenzungen (8, 9) umschlossenen Gehäuseinnenraumes (20) weisen.

17. Kniegelenkendoprothese nach einem der Ansprüche 1 bis 16, dadurch gekennzeichnet, daß das Gehäuse (6) auf seinen dem Gehäuseinnenraum (20) abgewandten Außenwandungen seiner seitlichen Begrenzungen (8, 9) die Flügel (25, 26) aufweist, von denen jeder in eine vom Gehäuseinnenraum (20) abgewandte Richtung weist und sich bogenförmig an einem unteren Rand des Gehäuses (6) erstreckt, der dem Tibiateil (1) zugewandt ist, in Richtung auf den die Flügel (25, 26) eine Gleitfläche (14) aufweisen.

18. Kniegelenkendoprothese nach einem der Ansprüche 1 bis 17, dadurch gekennzeichnet, daß die Flügel (25, 26) parallel zur Richtung der Schwenkachse (12) eine relativ große Breite aufweisen und einer aufdem Tibiateil (1) ausgebildeten Lagerfläche (38) in geringem Abstand gegenüberliegen.

19. Kniegelenkendoprothese nach einem der Ansprüche 1 bis 18, dadurch gekennzeichnet, daß die Gleitflächen (14) der beiden Flügel (25, 26) jeweils eine in Richtung auf den Tibiateil (1) ballig gewölbte schwache Neigung aufweisen, die sich vom Gehäuse (6) zu dem Gehäuse (6) gegenüberliegenden Außenkanten (80, 81) der Flügel (25, 26) erstreckt, und die Außenkanten (80, 81) mit Außenkanten (84, 85) kongruieren, die den Tibiateil (1) begrenzen.

20. Kniegelenkendoprothese nach Anspruch 19, dadurch gekennzeichnet, daß jeder Flügel (25, 26) sich über einen in etwa zur Schwenkachse (12) konzentrisch verlaufenden Bogen von etwa 180° an einer dem Tibiateil (1) zugewandten Unterkante (82) des Femurteils (2) erstreckt.

21. Kniegelenkendoprothese nach Anspruch 20, dadurch gekennzeichnet, daR jeder Flügel (25, 26) ein in Richtung auf den hinteren Teil (112) des Femurteils (2) weisendes hinteres Ende (83) aufweist, das von einer von der Horizontalebene (79) mit der Unterkante (82) des Gehäuses (6) gebildeten Schnittlinie (86) sich kreisbogenförmig konzentrisch zur Schwenkachse (12) bis zu einem knapp oberhalb der Horizontalebene (79) liegenden hinteren Ende (83) erstreckt.

22. Kniegelenkendoprothese nach Anspruch 21, dadurch gekennzeichnet, daß jeder Flügel (25, 26) sich entlang einem Bogen (87) vom hinteren Teil (83) zu einem diesem gegenüberliegenden vorderen Ende (88) erstreckt, das in einer dem hinteren Teil des Femurteils (2) gegenüberliegenden vorderen Abschlußfläche (89) des Gehäuses (6) liegt.

23. Kniegelenkendoprothese nach Anspruch 22, dadurch gekennzeichnet, daß das vordere Ende (88) um etwa eine gleiche Entfernung in Richtung auf den Tibiateil (1) unterhalb der Horizontalebene (79) liegt wie das hintere Ende (83) oberhalb dieser.

24. Kniegelenkendoprothese nach Anspruch 22 und 23, dadurch gekennzeichnet, daß der Bogen (87) vom hinteren Ende (83) zum vorderen Ende (88) verschiedene Krümmungsradien aufweist.

25. Kniegelenkendoprothese nach einem der Ansprüche 22 bis 24, dadurch gekennzeichnet, daß die vorderen Enden (88) der beiden Flügel (25, 26) über einen kurzen vom Tibiateil (1) abgewandten gewölbten Bogen (91) miteinander verbunden sind, der in einer das Gehäuse (6) begrenzenden vorderen Stirnwand (92) ausgebildet ist und einer entsprechenden Erhebung auf dem Tibiateil (1) entspricht.

26. Kniegelenkendoprothese nach einem der Ansprüche 1 bis 25, dadurch gekennzeichnet, daß das Tibiateil (1) im Bereich seiner dem Femurteil (2) zugewandten Ausdehnung einen vom Gehäuse (6) aufnehmbaren Steg (33) aufweist.

27. Kniegelenkendoprothese nach Anspruch 26, dadurch gekennzeichnet, daß der Steg (33) zwei einander etwa planparallel verlaufende Seitenwandungen (93, 94) aufweist, die jeweils in einem etwa gleich großen Abstand (95) von der sich mittig durch den Tibiateil (1) erstreckenden Beugeebene (96) angeordnet sind, planparallel zu dieser verlaufen und an Innenwandungen (98, 99) des Gehäuses (6) geführt sind.

28. Kniegelenkendoprothese nach Anspruch 27, dadurch gekennzeichnet, daß die Innenwandungen (98, 99) den Gehäuseinnenraum (20) begrenzen, der quer zur Beugeebene (96) einen im wesentlichen rechteckigen Querschnitt aufweist, in Richtung auf das vordere Ende (88) von der im wesentlichen planparallel zur Schaftebene (97) verlaufenden Abschlußfläche (89) begrenzt ist und in Richtung auf das hintere Ende (83) offen ist.

29. Kniegelenkendoprothese nach einem der Ansprüche 26 bis 28, dadurch gekennzeichnet, daß der Steg (33) sich mittig auf eine Auflageplatte (35) erhebt, die eine sich quer zur Längsachse (16) des Tibiateils (1) erstreckende Ebene aufspannt.

30. Kniegelenkendoprothese nach Anspruch 27, dadurch gekennzeichnet, daß der Steg (33) als eine Öse ausgebildet ist, deren dem Femurteil (2) zugewandte obere Begrenzung (100) als eine konzentrisch zur Ausnehmung (34) verlaufende Rundung ausgebildet ist.

31. Kniegelenkendoprothese nach Anspruch 29 und 30, dadurch gekennzeichnet, daß auf der Auflagefläche (35) eine Lagerschale (37) angeordnet ist, durch die der Steg (33) in Richtung auf den Femurteil (2) hindurchragt.

32. Kniegelenkendoprothese nach Anspruch 31, dadurch gekennzeichnet, daß die Lagerschale (37) den Steg (33) U-förmig umgreift und sich entlang den Seitenwandungen (93, 94) mit zwei Stegen (101, 102) erstreckt, die in einem der vorderen Abschlußfläche (89) zugewandten vorderen Teil (103) der Auflageplatte (35) durch ein Joch (104) miteinander verbunden sind und zwischen denen sich eine Öffnung (105) bis zu einem dem vorderen Teil (103) gegenüberliegenden hinteren Teil (106) der Auflageplatte (35) erstreckt, durch die sich auf dem hinteren Teil (106) der Steg (33) erhebt.

33. Kniegelenkendoprothese nach Anspruch 32, dadurch gekennzeichnet, daß die Lagerfläche (38) einen dem Joch (104) zugewandten vorderen Teil (107) aufweist, der vom vorderen Ende (88) der Flügel (25, 26) beaufschlagt und gegenüber einem in den hinteren Teil der Lagerschale (37) einmündenden flachen Teil (108) der Lagerfläche (38) in Richtung auf den Femurteil (2) angehoben ist.

34. Kniegelenkendoprothese nach Anspruch 33, dadurch gekennzeichnet, daß auf der Lagerfläche (38) die ihr zugewandte Gleitfläche (14) der Flügel (25, 26) aufliegt.

35. Kniegelenkendoprothese nach Anspruch 34, dadurch gekennzeichnet, daß die Lagerfläche (38) in ihrer quer zur Längsrichtung verlaufenden Querrichtung eine der Wölbung der Gleitfläche (14) angepaßte und die Flügel (25, 26) führende Wölbung aufweist, die in gestreckter Stellung im vorderen Teil (103) als eine Führung der Unterkante (82) der vorderen Stirnwand (92) ausgebildet ist.

36. Kniegelenkendoprothese nach einem der Ansprüche 30 bis 35, dadurch gekennzeichnet, daß in der Ausnehmung (34) eine Welle (5) befestigt ist, die als Scharniergelenk ausgebildet ist und in Lagerschalen (27, 28) drehbar gelagert ist, die im Gehäuse (6) befestigt sind.

37. Kniegelenkendoprothese nach Anspruch 36, dadurch gekennzeichnet, daß die Welle (5) eine Bohrung (44) aufweist, durch die und durch den Steg (33) sich eine die Welle (5) relativ zum Tibiateil (1) fixierende Madenschraube (46) erstreckt.

38. Kniegelenkendoprothese nach Anspruch 36 und 37, dadurch gekennzeichnet, daß die Welle (5) eine in Richtung der Schwenkachse (12) angeordnete und ihre Montage vereinfachende Bohrung (42) aufweist.

39. Kniegelenkendoprothese nach Anspruch 26 und 27, dadurch gekennzeichnet, daß der Steg (33) im wesentlichen U-förmig ausgebildet ist und eine dem Femurteil (2) zugewandt angeordnete Einführöffnung (49) aufweist.

40. Kniegelenkendoprothese nach Anspruch 39, dadurch gekennzeichnet, daß der Lagerzapfen (5) fest im Bereich des Gehäuses (6) gehaltert ist.

41. Kniegelenkendoprothese nach Anspruch 39 und 40, dadurch gekennzeichnet, daß das Tibiateil (1) und das Femurteil (2) in einem verschwenkten Zustand zusammenfügbar und bei einer Ausrichtung der Längsachsen (7, 16) in den Schaftebenen (97, 123) gegen in den Schaftebenen (97, 123) aufgebrachte Kräfte verblockt sind.

42. Kniegelenkendoprothese nach einem der Ansprüche 39 bis 41, dadurch gekennzeichnet, daß die Einführöffnung (49) von einem sich durch Schenkel (125, 126), die den U-förmigen Steg (33) begrenzen, erstreckenden Riegel (124) verschlossen ist.

43. Kniegelenkendoprothese nach Anspruch 42, dadurch gekennzeichnet, daß der Riegel (124) in den Schenkel (125, 126) formschlüssig geführt ist.

## Claims

1. A knee joint endoprosthesis comprising a femur member (2) and a tibia member (1) which are mounted pivotably in a bending plane (96) from an extended position into an angled position about a hinge joint which connects the femur member (2) and the tibia member (1) together and whose pivot axis (12) is at an invariable spacing both from the femur member (2) and also from the tibia member (1), the femur member and the tibia member each having respective shank portions (3, 4) which in the angled position include a bending angle (11) between them and in the extended position face in mutually opposite directions, wherein the femur member (2) has arcuate wings (25, 26) fixed thereto and extending through the shank portions (3, 4) are respective longitudinal axes (7, 16) which in the extended position are arranged substantially in shank portion planes (97, 123) which are in mutually plane-parallel relationship and which extend transversely with respect to the bending plane (96), wherein of the shank portion planes (7, 123) that of the femur member (2) is at a larger horizontal spacing (13) from a vertical plane (78) extending in plane-parallel relationship with them through the pivot axis (12) in a direction towards a rear portion (112) of the femur member (2), which rear portion is included by the bending angle (111), than the shank portion plane (97) of the tibia member (1) and wherein there is a shank portion spacing (122) between the shank portion plane (97) of the tibia member (1) and the shank portion plane (123) of the femur portion (2) and a horizontal plane (79) extending through the pivot axis (12) perpendicularly to the vertical plane (78) has, in a direction towards the tibia member (1), a vertical spacing (15) relative to a horizontal displacement plane (114) which extends tangentially relative to a support surface (117) provided on the tibia member (1), characterised in that the horizontal spacing (13) between the shank portion plane (123) of the femur member (2) and the vertical plane (78) is 16 to 21 mm, that the shank portion spacing (122) between the shank portion plane (97) of the tibia member (1) and the shank portion plane (123) of the femur member (2) at 0.5 to 4 mm is small in comparison with the horizontal spacings (13, 121) between the vertical plane (78) and the respective shank portion planes (97, 123), that the vertical spacing (15) is 20 to 24 mm and that with uniform loading of the two members (1, 2) the arcuate wings (25, 26) fixed to the femur member (2) slide on the support surface (117) which, with a one-sided loading of one of the two wings (26), is loaded under pressure on its surface which is in opposite relationship to the loaded wing (25, 26).

2. A knee joint endoprosthesis according to claim 1 characterised in that the shank portion plane (123) of the femur member (2) extends in plane-parallel relationship with the shank portion plane (97) of the tibia member (1) through the shank portion (3) of the tibia member (1).

3. A knee joint endoprosthesis according to claim 1 or claim 2 characterised in that the shank portion spacing (122) is 1.5 to 2.5 mm.

4. A knee joint endoprosthesis according to one of claims 1 to 3 characterised in that the horizontal spacing (13) is about 16 mm.

5. A knee joint endoprosthesis according to one of claims 1 to 4 characterised in that the vertical spacing (15) is about 21 mm.

6. A knee joint endoprosthesis according to one of claims 1 to 5 characterised in that at least one of the shank portions (3, 4) of the members (1, 2) is long in relation to the diameter thereof.

7. A knee joint endoprosthesis according to one of claims 1 to 6 characterised in that at least one of the shank portions (3, 4) is of a tapering configuration in the direction of the end remote from the other shank portion (3, 4).

8. A knee joint endoprosthesis according to one of claims 1 to 7 characterised in that at least one of the shank portions (3, 4) is free from any undercut configuration.

9. A knee joint endoprosthesis according to one of claims 1 to 8 characterised in that at least one of the shank portions (3, 4) is provided in the region of its end remote from the other shank portion with a centering star (47).

10. A knee joint endoprosthesis according to one of claims 1 to 9 characterised in that the centering star (49) is made from a plastics material.

11. A knee joint endoprosthesis according to one of claims 1 to 10 characterised in that the shank portions (3, 4) are of a quadrangular cross-section whose edges are rounded off.

12. A knee joint endoprosthesis according to one of claims 1 to 11 characterised in that arranged in the region of the enlargement of the femur member (2), which is towards the tibia member (1), is a housing (6) which substantially comprises two lateral boundary portions (8, 9) which extend in approximately mutually plane-parallel relationship and which extend from the shank portion (4) of the femur member (2) towards the tibia member (1).

13. A knee joint endoprosthesis according to one of claims 1 to 12 characterised in that arranged in the region of the lateral boundary portion (8, 9) of the housing (6) are bearing shells (27, 28) guiding a shaft (5) which extends transversely with respect to the bending plane (96) through the housing (6), a respective one of the bearing shells being mounted in a lateral boundary portion (8, 9).

14. A knee joint endoprosthesis according to one of claims 1 to 13 characterised in that one of the two bearing shells (27) is provided with a blind hole (29) for receiving the shaft (5).

15. A knee joint endoprosthesis according to one of claims 1 to 14 characterised in that the other bearing shell (28) has a through hole (31) which guides the shaft (5) and which is aligned with the blind hole (29).

16. A knee joint endoprosthesis according to one of claims 1 to 15 characterised in that recesses (10, 11) of the housing (6) each receive a respective shoulder (23, 24) of the bearing shells (27, 28) which lie with a respective outside surface (18, 19) on the lateral boundary portions (8, 9) and face in the direction of an inside space (20) of the housing, which is enclosed by the lateral boundary portions (8, 9).

17. A knee joint endoprosthesis according to one of claims 1 to 16 characterised in that on its outside walls, which are remote from the inside space (20) in the housing, of its lateral boundary portions (8, 9), the housing (6) has the wings (25, 26), each of which faces in a direction away from the inside space (20) in the housing and extends arcuately at a lower edge of the housing (6), which is towards the tibia member (1), in a direction towards which the wings (25, 26) have a sliding surface (14).

18. A knee joint endoprosthesis according to one of claims 1 to 17 characterised in that the wings (25, 26) are of a relatively great width parallel to the direction of the pivot axis (12) and are disposed at a small spacing in opposite relationship to a bearing surface (38) on the tibia member (1).

19. A knee joint endoprosthesis according to one of claims 1 to 18 characterised in that the sliding surfaces (14) of the two wings (25, 26) each involve a slight inclination which is spherically curved towards the tibia member (1) and which extends from the housing (6) to outside edges (80, 81) of the wings (25, 26), which are opposite to the housing (6), and the outside edges (80, 81) are congruent with outside edges (84, 85) which define the tibia member (1).

20. A knee joint endoprosthesis according to claim 19 characterised in that each wing (25, 26) extends over an arc of about 180° extending approximately concentrically with respect to the pivot axis (12) at a lower edge (82) of the femur member (2), which is towards the tibia member (1).

21. A knee joint endoprosthesis according to claim 20 characterised in that each wing (25, 26) has a rear end (83) which faces in a direction towards the rear portion (112) of the femur member (2) and which extends in an arcuate configuration concentrically with respect to the pivot axis (12) from an intersection line (86) formed by the horizontal plane (79) with the lower edge (82) of the housing (6) to a rear end (83) which is disposed just above the horizontal plane (79).

22. A knee joint endoprosthesis according to claim 21 characterised in that each wing (25, 26) extends along an arc (87) from the rear portion (83) to a front end (88) which is opposite thereto and which is disposed in a front terminal surface (89) of the housing (6), which is in opposite relationship to the rear part of the femur member (2).

23. A knee joint endoprosthesis according to claim 22 characterised in that the front end (88) lies at approximately an equal distance towards the tibia member (1) beneath the horizontal plane (79) as the rear end (83) above same.

24. A knee joint endosprosthesis according to claim 22 and claim 23 characterised in that the arc (87) from the rear end (83) to the front end (88) has different radii of curvature.

25. A knee joint endosprosthesis according to one of claims 22 to 24 characterised in that the front ends (88) of the two wings (25, 26) are connected together by way of a short curved arc (91) which faces away from the tibia member (1) and which is provided in a front end wall defining the housing (6) and corresponds to a corresponding raised portion on the tibia member (1).

26. A knee joint endosprosthesis according to one of claims 1 to 25 characterised in that in the region of its enlargement which is towards the femur member (2) the tibia member (1) has a limb portion(33) which can be received by the housing (6).

27. A knee joint endoprosthesis according to claim 26 characterised in that the limb portion (33) has two side walls (93, 94) which extend in approximately mutually plane-parallel relationship and which are each arranged at an approximately equal spacing (95) from the bending plane (96) extending centrally through the tibia member (1), extend in plane-parallel relationship therewith, and are guided at inside walls (98, 99) of the housing (6).

28. A knee joint endoprosthesis according to claim 27 characterised in that the inside walls (98, 99) define the space (20) inside the housing, which transversely with respect to the bending plane (96) is of a substantially rectangular cross-section, is delimited in a direction towards the front end (88) by the terminal surface (89) extending in substantially plane-parallel relationship with the shank portion plane (97), and is open in a direction towards the rear end (83).

29. A knee joint endoprosthesis according to one of claims 26 to 28 characterised in that the limb portion (33) rises centrally on the support plate (35) which defines a plane extending transversely with respect to the longitudinal axis (16) of the tibia member (1).

30. A knee joint endosprosthesis according to claim 27 characterised in that the limb portion (33) is in the form of an eye whose upper boundary (100) which is towards the femur member (2) is in the form of a rounded configuration extending concentrically with respect to the recess (34).

31. A knee joint endosprosthesis according to claim 29 and claim 30 characterised in that arranged on the support surface (35) is a bearing shell (37) through which the limb portion (33) projects in a direction towards the femur member (2).

32. A knee joint endoprosthesis according to claim 31 characterised in that the bearing shell (37) extends around the limb portion (33) in a U-shape and extends along the side walls (93, 94) with two limb portions (101, 102) which are connected together by a yoke (104) in a front part (103) of the support plate (35) that is towards the front terminal surface (89), and between which an opening (105) extends to a rear part (106) of the support plate (35) that is opposite to the front part (103), the limb portion (33) rising on the rear part (106) through the opening.

33. A knee joint endoprosthesis according to claim 32 characterised in that the bearing surface (38) has a front part (107) which is towards the yoke (104) and which is engaged by the front end (88) of the wings (25, 26) and which is raised towards the femur member (2) relative to a flat part (108) of the bearing surface (38) that opens into the rear part of the bearing shell (37).

34. A knee joint endoprosthesis according to claim 33 characterised in that the sliding surface (14) of the wings (25, 26), that is towards the bearing surface (38), rests thereon.

35. A knee joint endoprosthesis according to claim 34 characterised in that in its transverse direction which extends transversely with respect to the longitudinal direction the bearing surface (38) has a curvature which is adapted to the curvature of the sliding surface (14) and which guides the wings (25, 26) and which in the extended position is formed in the front part (103) as a guide for the lower edge (82) of the front end wall (92).

36. A knee joint endoprosthesis according to one of claims 30 to 35 characterised in that fixed in the recess (34) is a shaft (5) which is in the form of a hinge joint and which is rotatably mounted in bearing shells (27, 28) which are fixed in the housing (6).

37. A knee joint endoprosthesis according to claim 36 characterised in that the shaft (5) has a bore (44) through which and through the limb portion (33) extends a grub screw (46) which fixes the shaft (5) relative to the tibia member (1).

38. A knee joint endoprosthesis according to claim 36 and claim 37 characterised in that the shaft (5) has a bore (42) which is arranged in the direction of the pivot axis (12) and which simplifies assembly thereof.

39. A knee joint endoprosthesis according to claim 26 and claim 27 characterised in that the limb portion (33) is of substantially U-shaped configuration and has an insertion opening (49) which is arranged facing towards the femur member (2).

40. A knee joint endoprosthesis according to claim 39 characterised in that the bearing pin (5) is held fast in the region of the housing (6).

41. A knee joint endoprosthesis according to claim 39 and claim 40 characterised in that the tibia member (1) and the femur member (2) can be fitted together in a pivoted condition and are blocked against forces applied in the shank portion planes (97, 123) upon alignment of the longitudinal axes (7, 16) in the shank portion planes (97, 123).

42. A knee joint endoprosthesis according to one of claims 39 to 41 characterised in that the insertion opening (49) is closed by a lock member (124) extending through legs (125, 126) which define the U-shaped limb portion (33).

43. A knee joint endoprosthesis according to claim 42 characterised in that the lock member (124) is positively lockingly guided in the legs (125, 126).

## Revendications

1. Endoprothèse d'articulation de genou comportant un élément fémoral (2) et un élément tibial (1) qui sont montés avec possibilité de pivotement d'une position étendue dans une position pliée dans un plan de flexion (96), autour d'une articulation à charnière reliant l'un à l'autre les des éléments, dont l'axe de pivotement (12) est disposé à une distance non modifiable de l'élément fémoral (2) et de l'élément tibial (1), et qui présentent chacun des tiges (3, 4) qui, dans la position pliée, forment entre elles un angle de flexion (11) et dans la position étendue sont orientées dans des directions opposées, l'élément fémoral (2) présentant des ailes (25, 26) en forme d'arcs qui sont fixées audit élément et un ce longitudinal (7, 16) s'étendant à travers chacune des tiges (3, 4), axes qui dans la position étendue sont disposés dans des plans de tige (97, 123) sensiblement mutuellement parallèles qui s'étendent transversalement au plan de flexion (96), parmi les plans de tige (97, 123), le plan de tige de l'élément fémoral (2) étant situé à une distance horizontale (13) plus grande que le plan de tige (97) de l'élément tibial (1) par rapport à un plan vertical (78) parallèle auxdits plans passant par l'axe de pivotement (12) en direction d'une partie postérieure (112) de l'élément fémoral (2) incluse dans l'angle de flexion (111), une distance entre tiges (122) existant entre le plan de tige (97) de l'élément tibial (1) et le plan de tige (123) de l'élément fémoral (2) et un plan horizontal (79) qui est perpendiculaire au plan vertical (78) et passe par l'axe de pivotement (12) étant situé à une distance verticale (15) en direction de l'élément tibial (1) par rapport à un plan de glissement (114) horizontal qui s'étend tangentiellement à une surface d'appui (117) aménagée sur l'élément tibial (1), caractérisée par le fait que la distance horizontale (13) entre le plan de tige (123) de l'élément fémoral (2) et le plan vertical (78) est comprise dans une plage allant de 16 à 21 mm, par le fait que la distance entre tiges (122) entre le plan de tige (97) de l'élément tibial (1) et le plan de tige (123) de l'élément fémoral (2) comprise entre 0,5 et 4 mm est petite par rapport aux distances horizontales (13, 121) entre le plan vertical (78) et les plans de tige (97, 123) concernés, par le fait que la distance verticale (15) est comprise dans une plage allant de 20 à 24 mm et par le fait qu'en présence d'une sollicitation uniforme des deux éléments (1, 2) les ailes arquées (25, 26) fixées à l'élément fémoral (2) glissent sur la surface d'appui (117) qui, lors d'une charge unilatérale de l'une des deux ailes (25, 26), est sollicitée en compression au niveau de sa surface opposée à l'aile (25, 26) chargée.

2. Endoprothèse d'articulation de genou selon la revendication 1, caractérisée par le fait que le plan de tige (123) de l'élément fémoral (2) s'étend parallèlement au plan de tige (97) de l'élément tibial (1) à travers la tige (3) de l'élément tibial (1).

3. Endoprothèse d'articulation de genou selon la revendication 1 ou 2, caractérisée par le fait que la distance entre tiges (122) est comprise dans une plage allant 1,5 & 2,5 mm.

4. Endoprothèse d'articulation de genou selon une des revendications 1 à 3, caractérisée par le fait que la distance horizontale (13) est d'environ 16 mm.

5. Endoprothèse d'articulation de genou selon une des revendications 1 à 4, caractérisée par le fait que la distance verticale (15) est d'environ 21 mm.

6. Endoprothèse d'articulation de genou selon une des revendications 1 à 5, caractérisée par le fait qu'au moins une des tiges (3, 4) des éléments (1, 2) est longue par rapport à son diamètre.

7. Endoprothèse d'articulation de genou selon une des revendications 1 à 6, caractérisée par le fait qu'au moins une des tiges (3, 4) se rétrécit en direction de son extrémité éloignée de l'autre tige (3, 4).

8. Endoprothèse d'articulation de genou selon une des revendications 1 à 7, caractérisée par le fait qu'au moins une des tiges (3, 4) est réalisée sans contre-dépouille.

9. Endoprothèse d'articulation de genou selon une des revendications 1 à 8, caractérisée par le fait qu'au moins une des tiges (3, 4) est pourvue d'une étoile de centrage (47) dans la région de son extrémité éloignée de l'autre tige.

10. Endoprothèse d'articulation de genou selon une des revendications 1 à 9, caractérisée par le fait que l'étoile de centrage (47) est réalisée en une matière plastique.

11. Endoprothèse d'articulation de genou selon une des revendications 1 à 10, caractérisée par le fait qu'au moins une des tiges (3, 4) présente une section quadrangulaire dont les angles sont arrondis.

12. Endoprothèse d'articulation de genou selon une des revendications 1 à 11, caractérisée par le fait qu'un boîtier (6) est disposé dans la région de l'extension de l'élément fémoral (2) tournée vers l'élément tibial (1), lequel boîtier est essentiellement formé de deux parois latérales (8, 9) mutuellement parallèles qui s'étendent depuis la tige (4) de l'élément fémoral (2) en direction de l'élément tibial (1).

13. Endoprothèse d'articulation de genou selon une des revendications 1 à 12, caractérisée par le fait que des coquilles de palier (27, 28) assurant le guidage d'un axe (5) qui s'étend à travers le boîtier (6) transversalement au plan de flexion (96), sont disposées dans la région des parois (8, 9) du boîtier (6), lesquelles coquilles de palier sont montées chacune dans une paroi latérale (8, 9).

14. Endoprothèse d'articulation de genou selon une des revendications 1 à 13, caractérisée par le fait que l'une des deux coquilles de palier (27) est pourvue d'un trou non débouchant (29) qui reçoit l'axe (5).

15. Endoprothèse d'articulation de genou selon une des revendications 1 à 14, caractérisée par le fait que l'autre coquille de palier (28) présente un trou débouchant (31) qui assure le guidage l'axe (5) et est aligné avec le trou non débouchant (29).

16. Endoprothèse d'articulation de genou selon une des revendications 1 à 15, caractérisée par le fait que des évidements (10, 11) du boîtier (6) reçoivent chaque fois un collet (23, 24) des coquilles de palier (27, 28) qui repose par une surface extérieure (18, 19) sur les parois latérales (8, 9) et est dirigé vers une chambre intérieure de bôitier (20) entourée par les parois latérales (8, 9).

17. Endoprothèse d'articulation de genou selon une des revendications 1 à 16, caractérisée par le fait que le boîtier (6) présente sur ses parois extérieures éloignées de la chambre intérieure de boîtier (20) de ses parois latérales (8, 9) les ailes (25, 26) qui sont orientées chacune dans une direction opposée à la chambre intérieure de boîtier (20) et s'étendent en arc de cercle sur un bord inférieur du boîtier (6) tourné vers l'élément tibial (1), en direction duquel les ailes (25, 26) présentent une surface de glissement (14).

18. Endoprothèse d'articulation de genou selon une des revendications 1 à 17, caractérisée par le fait que parallèlement à la direction de l'axe de pivotement (12), les ailes (25, 26) présentent une largeur relativement grande et sont disposées en vis-à-vis d'une surface de palier (38) aménagée sur l'élément tibial (1), à faible distance de celle-ci.

19. Endoprothèse d'articulation de genou selon une des revendications 1 à 18, caraetérisée par le fait que les surfaces de glissement (14) des deux ailes (25, 26) présentent chacune une faible déclivité bombée sphérique en direction de l'élément tibial (1), qui s'étend depuis le boîtier (6) jusqu'aux bords extérieurs (80, 81) des ailes en vis-à-vis du boîtier (6) et que les bords extérieurs (80, 81) sont congruents avec des bords extérieurs (84, 85) qui limitent l'élément tibial (1).

20. Endoprothèse d'articulation de genou selon la revendication 19, caractérisée par le fait que chaque aile (25, 26) s'étend sur un arc d'environ 180° sensiblement concentrique à l'axe de pivotement (12) sur un bord inférieur (82) de l'élément fémoral (2) tourné vers l'élément tibial (1).

21. Endoprothèse d'articulation de genou selon la revendication 20, caractérisée par le fait que chaque aile (25, 26) présente une extrémité postérieure (83) orientée en direction de la partie postérieure (112) de l'élément fémoral (2), qui s'étend en arc de cercle de manière concentrique à l'axe de pivotement (12), depuis une ligne d'intersection (86) formée par le plan horizontal (79) et le bord inférieur (82) du boîtier (6) jusqu'à une extrémité postérieure (83) située juste au dessus du plan horizontal (79).

22. Endoprothèse d'articulation de genou selon la revendication 21, caractérisée par le fait que chaque aile (25, 26) s'étend le long d'un arc (87) depuis la partie postérieure (83) jusqu'à une partie antérieure (88) en vis-à-vis, qui est située dans une surface terminale (89) antérieure du boîtier (6) en vis-à-vis de la partie postérieure de l'élément fémoral (2).

23. Endoprothèse d'articulation de genou selon la revendication 22, caractérisée par le fait que l'extrémité antérieure (88) est située sensiblement à une même distance en direction de l'élément tibial (1) au-dessous du plan horizontal (79) que l'extrémité postérieure (83) est situé au-dessus dudit plan.

24. Endoprothèse d'articulation de genou selon les revendications 22 et 23, caractérisée par le fait que l'arc (87) présente différents rayons de courbure de l'extrémité postérieure (83) jusqu'à l'extrémité antérieure (88).

25. Endoprothèse d'articulation de genou selon une des revendications 22 à 24, caractérisée par le fait que les extrémités antérieures (88) des deux ailes (25, 26) sont reliées l'une à l'autre par un arc (91) de faible longueur incurvé dans la direction opposée à l'élément tibial (1), qui est aménagé dans une paroi antérieure (92) délimitant le boîtier (6) et qui correspond à une surélévation correspondante sur l'élément tibial (1).

26. Endoprothèse d'articulation de genou selon une des revendications 1 à 25, caractérisée par le fait que l'élément tibial (1), dans la région de son extension tournée vers l'élément fémoral (2), présente une cloison (33) qui est reçue dans le boîtier (6).

27. Endoprothèse d'articulation de genou selon la revendication 26, caractérisée par le fait que la cloison (33) présente deux parois latérales (93, 94) sensiblement mutuellement parallèles qui sont disposées chacune à une distance (95) sensiblement égale du plan de flexion (96) passant par le milieu de l'élément tibial (1), s'étendent parallèlement audit plan et sont guidées sur des parois intérieures (98, 99) du boîtier (6).

28. Endoprothèse d'articulation de genou selon la revendication 27, caractérisée par le fait que les parois intérieures (98, 99) délimitent la chambre intérieure du boîtier (20) qui, transversalement au plan de flexion (96), présente une section sensiblement rectangulaire, est délimitée par la surface terminale (89) sensiblement parallèle au plan de tige (97) dans la direction de l'extrémité antérieure (88) et est ouverte dans la direction de l'extrémité postérieure (83).

29. Endoprothèse d'articulation de genou selon une des revendications 26 à 28, caractérisée par le fait que la cloison (33) fait saillie au milieu d'une plaque d'appui (35) qui définit un plan qui s'étend transversalement à l'axe longitudinal (16) de l'élément tibial (1).

30. Endoprothèse d'articulation de genou selon la revendication 27, caractérisée par le fait que la cloison (33) est conformée en oeillet dont la paroi supérieure (100) tournée vers l'élément fémoral (2) est agencée sous la forme d'un arrondi concentrique à l'évidement (34).

31. Endoprothèse d'articulation de genou selon les revendications 29 et 30, caractérisée par le fait qu'une coquille de palier (37) à travers laquelle la cloison (33) s'étend en direction de l'élément fémoral (2) est disposée sur la surface d'appui (35).

32. Endoprothèse d'articulation de genou selon la revendication 31, caractérisée par le fait que la coquille de palier (37) entoure en forme de U la cloison (33) et s'étend le long des parois latérales (93, 94) avec deux ailes (101, 102) qui, dans une partie antérieure (103) de la plaque d'appui (35) tournée vers la surface terminale antérieure (89) sont liées l'une à l'autre par une semelle (104) et entre lesquelles une ouverture (105) dans laquelle s'engage la cloison (33) sur la partie postérieure (106) s'étend jusqu'à une partie postérieure (106) de la plaque d'appui (35) en vis-à-vis de la partie antérieure (103).

33. Endoprothèse d'articulation de genou selon la revendications 32, caractérisée par le fait que la surface de palier (38) présente une partie antérieure (107) tournée vers la semelle (104) qui est sollicitée par l'extrémité antérieure (88) des ailes (25, 26) et qui par rapport à une partie plane (108) de la surface de palier (38) débouchant da,s la partie postérieure de la coquille de palier (37), est surélevée en direction de l'élément fémoral (2).

34. Endoprothèse d'articulation de genou selon la revendications 33, caractérisée par le fait que la surface de glissement (14) des ailes (25, 26) tournée vers la surface de palier (38) repose sur ladite surface de palier.

35. Endoprothèse d'articulation de genou selon la revendication 34, caractérisée par le fait que la surface de palier (38), dans la direction transversale par rapport à la direction longitudinale présente une partie bombée adaptée à la partie bombée de la surface de glissement (14) qui guide les ailes (25, 26) et qui dans la position étendue, dans la partie antérieure (103), est conformée en guide pour le bord inférieur (82) de la paroi frontale antérieure (92).

36. Endoprothèse d'articulation de genou selon une des revendications 30 à 35, caractérisée par le fait qu'un axe (5) est fixé dans l'évidement (34), lequel axe est agencé en articulation de charnière et est monté tournant dans des coquilles de palier (27, 28) fixées au boîtier (6).

37. Endoprothèse d'articulation de genou selon la revendication 36, caractérisée par le fait que l'axe (5) présente un trou (44) et qu'une vis sans tête (46) qui immobilise l'axe (5) par rapport à l'élément tibial (1) s'étend à travers ledit trou et à travers la cloison (33).

38. Endoprothèse d'articulation de genou selon les revendications 36 et 37, caractérisée pu le fait que l'axe (5) présente un trou (42) qui est disposé dans la direction de l'axe de pivotement (12) et facilite son montage.

39. Endoprothèse d'articulation de genou selon les revendications 26 et 27, caractérisée par le fait que la cloison (33) a essentiellement la forme d'un U et présente une ouverture d'entrée (49) tournée vers l'élément fémoral (2).

40. Endoprothèse d'articulation de genou selon la revendication 39, caractérisée par le fait que le tourillon (5) est tenu fermement dans la région du boîtier (6).

41. Endoprothèse d'articulation de genou selon les revendications 39 et 40, caractérisée par le fait que l'élément tibial (1) et l'élément fémoral (2) peuvent être assemblés l'un avec l'autre dans une position pivotée et sont bloqués vis-à-vis de forces appliquées dans les plans de tiges (97, 123) lorsque les axes longitudinaux (7, 16) sont alignés dans les plans do tiges (97, 123).

42. Endoprothèse d'articulation de genou selon une des revendications 39 à 41, caractérisée par le fait que l'ouverture d'entrée (49) est fermée par un verrou (124) qui s'étend à travers des ailes (125, 126) délimitant la cloison en forme de U (33).

43. Endoprothèse d'articulation de genou selon la revendication 42, caractérisée par le fait que le verrou (124) est guidé par obstacle dans les ailes (125, 126).
